# EUROPEAN PATENT SPECIFICATION

(11) **EP 2 861 990 B1**
(45) Date of publication and mention of the grant of the patent: **14.11.2018**
(21) Application number: 13747512.5
(22) Date of filing: 14.06.2013
(51) Int. Cl.: G01N 33/574

(54) **A METHOD AND A KIT FOR THE IN VITRO DIAGNOSIS OF PANCREATIC DUCTAL ADENOCARCINOMA OR FOR DETERMINING THE PREDISPOSITION TO PANCREATIC DUCTAL ADENOCARCINOMA**
VERFAHREN UND KIT ZUR IN-VITRO-DIAGNOSE VON DUKTALEM ADENOKARZINOM DES PANKREAS ODER ZUR BESTIMMUNG DER PRÄDISPOSITION FÜR DUKTALES ADENOKARZINOM DES PANKREAS
MÉTHODE ET TROUSSE POUR LE DIAGNOSTIC IN VITRO D'UN ADÉNOCARCINOME DU CONDUIT PANCRÉATIQUE OU POUR LA DÉTERMINATION DE LA PRÉDISPOSITION À UN ADÉNOCARCINOME DU CONDUIT PANCRÉATIQUE

(30) Priority: 15.06.2012 IT TO20120523
(43) Date of publication of application: 22.04.2015
(73) Proprietor: Natimab Therapeutics S.r.l., 10010 Colleretto Giacosa (Torino) (IT)
(72) Inventor: NOVELLI, Francesco, I-10136 Torino (IT); CAPPELLO, Paola, I-10125 Torino (IT); CAPELLO, Michela, I-10041 Carignano (Torino) (IT)
(74) Representative: Comoglio, Elena
(86) International application number: PCT/IB2013/054876
(87) International publication number: WO 2013/186748

(56) References cited:
- WO-A2-2006/015079
- WO-A2-2012/129192
- JP-A- 2008 309 612
- US-A1- 2008 318 225
- FELIX RÜCKERT ET AL: "Five Primary Human Pancreatic Adenocarcinoma Cell Lines Established by the Outgrowth Method", JOURNAL OF SURGICAL RESEARCH, ACADEMIC PRESS INC., SAN DIEGO, CA, US, vol. 172, no. 1, 8 April 2011 (2011-04-08) , pages 29-39, XP028336944, ISSN: 0022-4804, DOI: 10.1016/J.JSS.2011.04.021 [retrieved on 2011-04-14]
- ANGELA Y. WEHR ET AL: "Relative Quantification of Serum Proteins from Pancreatic Ductal Adenocarcinoma Patients by Stable Isotope Dilution Liquid Chromatography-Mass Spectrometry", JOURNAL OF PROTEOME RESEARCH, vol. 11, no. 3, 2 March 2012 (2012-03-02), pages 1749-1758, XP055052056, ISSN: 1535-3893, DOI: 10.1021/pr201011f
- CUI Y ET AL: "Expression of Ezrin and Phosphorylated Ezrin (pEzrin) in Pancreatic Ductal Adenocarcinoma", CANCER INVESTIGATION, vol. 28, no. 3, 1 March 2010 (2010-03-01), pages 242-247, XP008164316, DOI: 10.3109/07357900903124498

## Description

The present invention relates to the field of diagnosis of tumor pathologies, more specifically pancreatic ductal adenocarcinoma, also referred to as ACP.

Ductal adenocarcinoma (ACP) is the most frequent pancreatic tumor and represents the fourth cause of death in the United States and Europe. In its advanced stage, pancreatic adenocarcinoma has a very poor diagnosis with an average survival time of four months and a 2% five-year survival rate from diagnosis.

The extremely negative prognosis of this disease mainly depends on the high proliferative and invasive ability that characterizes pancreatic tumor cells, as well as the frequent delay in diagnosis caused by, in most cases, the lack of specific symptoms in the early stage of the disease. The first symptoms of the disease, often limited to loss of appetite and weight, are frequently underestimated, leading to a lethal delay in diagnosis of the tumor, generally at a stage when the tumor mass is no longer completely excisable.

Despite the considerable efforts made in the oncology therapy field, to date surgical resection remains the major treatment for patients affected by ACP, and the results from several clinical studies demonstrated a direct correlation between the disease prognosis following pancreatectomy and the early stage of the tumor at the time of surgery. On the whole, the 5-year survival in resected patients is 10-20%, but it increases to 30-60% for excised tumors of less than 2 cm in diameter or even to 70% for tumors of less than 1 cm in diameter (Sohn TA et al,. J Gastrointest Surg 2000 4(6): 567-79).

On the basis of the above discussion, therefore there is an urgency to provide suitable methods for the early diagnosis of pancreatic ductal adenocarcinoma, from the first stages of neoplastic transformation, in order to be able to intervene rapidly, thus significantly increasing the patients' survival likelihood.

A method for measuring the level of ezrin (aka villin-2) in human serum from pancreatic ductal adenocarcinoma patients is disclosed in International patent application WO 2006/015079.

A method of diagnosing ovarian cancer comprising the step of detecting autoantibodies against ezrin is disclosed in JP 2008 309612.

Also serological tests fall within the usual diagnosis of ACP, which are mainly based on assessment of the carbohydrate antigen marker 19.9 (CA 19.9), conventionally used as an indicator of the presence and seriousness of the tumor. CA 19.9 antigen is a membrane glycoprotein found in blood at very low concentrations under physiological conditions; in the course of the neoplastic disease the levels of this protein in serum increase significantly, as a result of changes in the processes that regulate mucin production and entry in the circulation. However, recent studies revealed that assessment of the CA 19.9 antigen does not allow to diagnose the pancreatic tumor in its early phases, as the blood levels of this protein correlate with the size of the tumor mass and are difficult to measure in patients with tumors with a diameter smaller than 3 cm. Additionally, the significance of the CA 19.9 antigen as a diagnostic marker of ACP is further compromised by the fact that it is possible to detect a substantial increase in the serum level thereof even in the course of other diseases of the digestive tract, including acute and chronic pancreatitis, chronic inflammatory bowel disease, and benign jaundice. In corroboration of the above discussion, several associations of persons of skill in the art, such as the European Group on Tumour Markers, the National Academy of Clinical Biochemistry (NACB), and the National Cancer Comprehensive Network (Klapdor R, Aronsson AC, Duffy MJ, et al. Tumor markers in gastrointestinal cancers, EGTM recommendations. Anticancer Res 1999; 19: 2785-2820) recommend the diagnostic use of the CA19.9 marker only as a complementary test and support for the customary radiological procedures.

The researches described in Tomaino B, et al. CIRCULATING AUTOANTIBODIES TO PHOSPHORYLATED ALPHA-ENOLASE ARE A HALLMARK OF PANCREATIC CANCER. J Proteome Res. 2011; 10: 105-112 on the serum proteomic profiles of a large cohort of patients affected by ACP, and relevant controls, revealed, in 62% of neoplasias, the presence of circulating auto-antibodies directed to particular isoforms of the glycolytic enzyme alpha-enolase, more specifically isoforms that are phosphorylated on serine at position 419 (ENOA1-2). By contrast, the above immunoreactivity was detectable only in 4% of control samples. As a further support to the clinical importance of these results, the authors demonstrated that in most cases the antibody response to the phosphorylated alpha-enolase isoforms correlates with a more favorable prognosis of the disease and a significant increase in estimated survival.

The use of the human phosphorylated alpha-enolase isoform as a biomarker in the diagnosis of APC, together with isolated peptides derived therefrom and containing the phosphorylation site, and with antibodies capable of specifically binding the phosphorylated epitope, is described in the Italian Patent Application TO2009000697.

In spite of the fact that, on the basis of the foregoing, the assessment of auto-antibodies directed to the phosphorylated ENOA 1,2 epitope has an important diagnostic value, particularly a prognostic one, its application in the field of early diagnosis of pancreatic carcinoma is still limited. Actually, in patients bearing a resectable tumor, and thus in an early stage of the disease, it has been verified that the presence of auto-antibodies against phosphorylated enolase is able to distinguish the pancreatic neoplasia with a sensitivity not greater than 61%.

Therefore, the present invention has the object of providing a method for the diagnosis of pancreatic ductal adenocarcinoma which overcomes the disadvantages and limitations of the prior art.

More particularly, an object of the present invention is to provide a diagnostic method that allows to identify with a high degree of accuracy patients affected by ACP at an early stage of the disease, and thus still surgically and/or therapeutically treatable with a significant margin of curative success.

This and other objects are attained by the *in vitro* method for the diagnosis of pancreatic ductal adenocarcinoma or for determining the predisposition to pancreatic ductal adenocarcinoma, as defined in the appended claim 1, and by the relevant kits, as defined in appended claims 9 and 10. Preferred embodiments of the method and kits of the invention are defined in the dependent claims.

The appended independent and dependent claims are an integral part of the present description.

As will be illustrated in more detail in the experimental section that follows, the present inventors found out that serum samples collected from patients affected by pancreatic carcinoma display a significant increase in the levels of circulating auto-antibodies against the human protein ezrin compared to control samples from healthy subjects or patients affected by different pathologies, such as non-pancreatic tumors, autoimmune diseases, or chronic pancreatitis.

In particular, serological examinations carried out on a cohort of patients representing the different developmental stages of ACP revealed that immunoreactivity against ezrin is present at high levels since the earliest stages of the tumor growth. The presence of autologous anti-ezrin antibodies in clinical samples then represents an ideal biomarker in that it is specific and selective for pancreatic tumor in its different developmental stages, allowing for the setting up of diagnostic methods targeted to the identification of patients in an early stage of the oncologic disease, for whom the quickness of the surgical and/or therapeutic intervention significantly correlates with the subsequent chances of survival.

Therefore, an object of the present invention is an *in vitro* method for the diagnosis of pancreatic ductal adenocarcinoma or for determining the predisposition of a subject to pancreatic ductal adenocarcinoma, comprising the step of determining, in a biological sample from the subject, the presence, the absence or the level of antibodies against ezrin, wherein the presence of antibodies against ezrin or the level of antibodies against ezrin above a predetermined threshold is indicative of pancreatic ductal adenocarcinoma or of a predisposition to pancreatic ductal adenocarcinoma.

According to one embodiment, the subject is a human patient suspected of being affected by pancreatic ductal adenocarcinoma.

According to another embodiment, the subject is a human being whose risk of developing pancreatic ductal adenocarcinoma or predisposition to pancreatic ductal adenocarcinoma one desires to determine.

Ezrin is a protein belonging to the family of ERM molecules (ezrin, radixin, and moesin), which mainly play the role of mediating the link between transmembrane cell receptors and adhesion molecules with actin filaments that make up the cytoskeleton. Ezrin in its inactive form resides in the cytoplasm, but, subsequent to phosphorylation-mediated activation, translocates to the apical cell membrane where it binds the cortical actin filaments and takes part in Rho and PI3K/Akt signal transduction pathways (Fehon RG et al.; 2010 Nat Rev Mol Cell Biol. 11(4): 276-87). Under physiological conditions, the ezrin protein is essentially involved in the macropinocytosis, cell adhesion, and membrane wrinkling mechanisms characteristic of epithelial cells, while it can take on a key role in the regulation of metastatic processes in the course of neoplastic diseases (Niggli V. et al; 2008 Int J Biochem Cell Biol 40(3): 344-9). Recent studies demonstrated an increase in ezrin protein expression levels in many tumor types.

The experimental route that led the present inventors to the identification of the specific association between the antibody response to ezrin and pancreatic ductal adenocarcinoma, which will be described in more detail in the experimental section, in a first phase used particularly sophisticated murine ACP models, based on genetically modified animals (GEM) in which mutated Kras and p53 alleles were introduced (S. R. Hingorani et al., 2003 Cancer Cell 4, 437; S. R. Hingorani et al., 2005 Cancer Cell 7, 469-83). More specifically, at first the conditioned expression of the mutated Kras gene (G12D) induces in these animals the formation of pancreatic intraepithelial neoplasias (PanIN) that, thanks to the concerted action of p53 gene mutations (R172H), progress quickly through the subsequent developmental stages of the tumor up to the invasive carcinoma one. By virtue of the above description, the murine GEM models are able to develop pancreatic ductal adenocarcinoma through specific pre-malign lesions and subsequent phases of neoplastic progression, thereby recapitulating the human tumor and allowing the study thereof from the initial stages.

In order to specifically assess the humoral response evoked in GEM mice by ACP development, the present inventors employed a comparative proteomic approach based on analysis of immunoreactivity profiles in serum samples collected from the animals at different developmental stages of the pancreatic tumor, and from relevant controls, when tested on protein extracts from murine pancreatic tumor cells previously separated by two-dimensional electrophoresis (2-DE). Such an approach allowed to detect in GEM animals, compared to the control group, a significant antibody response to the ezrin antigen as early as the age of one month, when the neoplastic progression is still at the precancerous lesion stage.

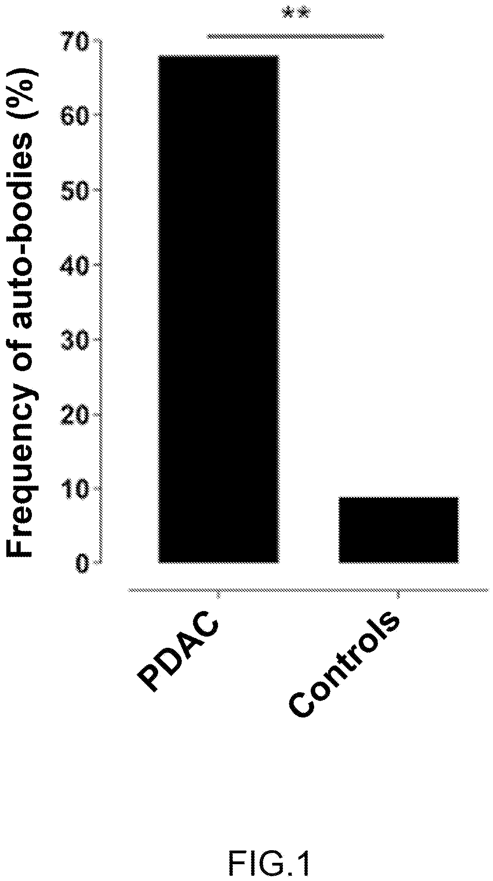

In the subsequent validation phase, the results from the proteomic analysis carried out on the murine models were confirmed by testing the levels of circulating anti-ezrin auto-antibodies in human serum samples collected from patients affected by ACP both at an advanced stage and at a stage in which the tumor was still resectable (stages II and III), and from relevant control groups. As illustrated by the graph in Figure 1, the antibody response to ezrin measured in sera from resected patients shows a frequency of about 68% when the samples are tested on protein extracts obtained from the human pancreatic tumor cell line CF-PAC-1, previously subjected to two-dimensional electrophoresis.

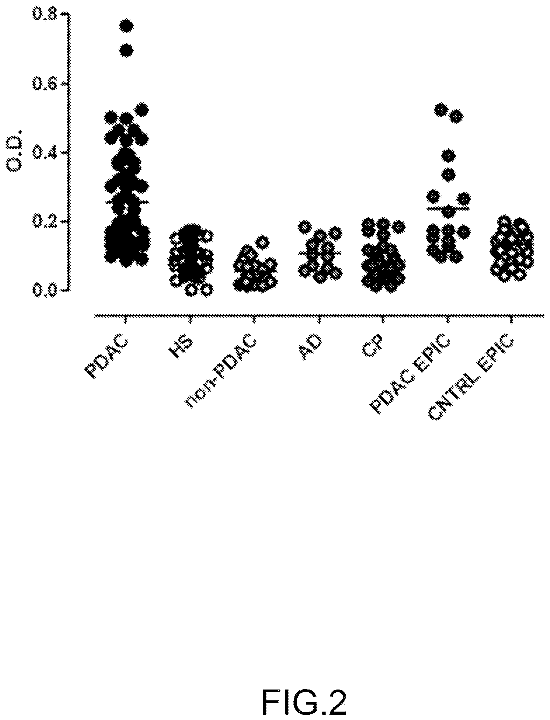

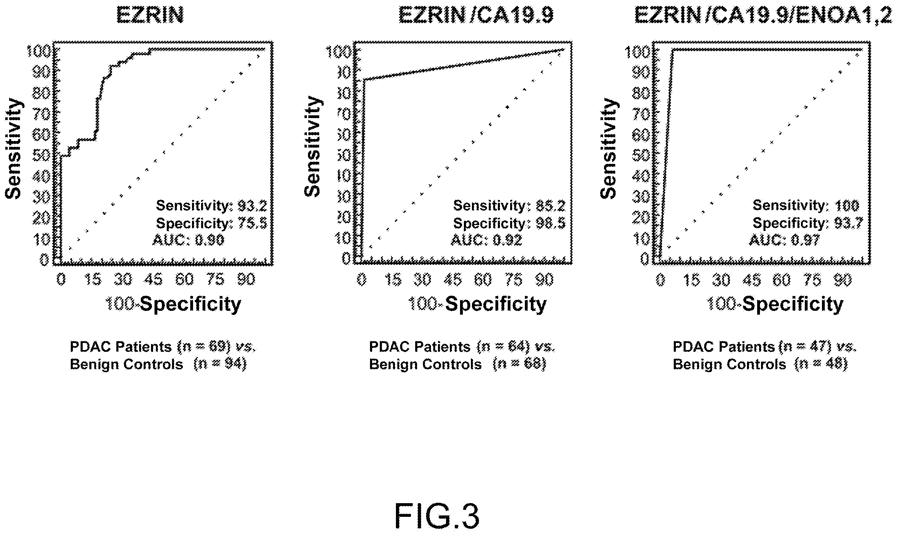

Further supporting the previously described experimental results, the present inventors checked the diagnostic value of the anti-ezrin auto-antibodies in an ELISA immunoassay, by using human recombinant ezrin protein as the affinity reagent. Particularly, a comparative analysis was carried out on the levels of immunoreactivity against this protein measured in serum samples from patients bearing a pancreatic carcinoma, both at a still operable stage and at an advanced stage, and in a control cohort comprising healthy subjects (HS) and patients affected by non-pancreatic tumors (non-PDAC), autoimmune diseases (AD) or chronic pancreatitis (CP). In order to attain a higher degree of completeness of the subject study, serum samples collected in the course of the extensive prospective multicentric study EPIC were also subjected to an ELISA assay, in which study, during a long follow-up that went on till 117 months from the first sampling, the incidence of pancreatic carcinoma onset was assessed in subjects enrolled at the beginning of the study in ten different European countries in the absence of the disease or of any relevant symptom. The results from the ELISA test, illustrated by the scatter plot in Figure 2, only demonstrate the presence of a specific reactivity towards the ezrin protein in sera from patients affected by pancreatic ductal adenocarcinoma and in "EPIC" subjects who developed this neoplasia during the study follow-up period (PDAC-EPIC). Vice versa, no immunoreactivity was detected in control samples and in "EPIC" samples from subjects unaffected by pancreatic tumor at the end of follow-up (CNTRL-EPIC). ROC analysis showed that a cut-off of 0.1183 optical density arbitrary units distinguishes patients affected by pancreatic carcinoma from control subjects with a sensitivity of 93.2%, a specificity of 75.5%, area under the curve (AUC) 0.90±0.03 (Figure 3A). Moreover, the discriminating ability of the diagnostic method of the invention undergoes a significant increase (sensitivity of 100%, specificity of 80.3%, AUC 0.95) when the serological test is restricted to samples collected from patients affected by pancreatic tumor at a still resectable stage, thus proving its value in the course of early investigations. By contrast, the diagnostic accuracy exclusively based on the use of the customary biomarker CA 19.9 on the same samples revealed an appreciable decrease indicated by a 0.85 AUC value of the ROC curve.

Any type of biological sample suitable for assaying the protein content can be used for carrying out the diagnostic method of the invention, preferably blood samples and derivatives thereof, such as serum and plasma.

In the clinical-diagnostic practice, the use of combinations of biomarkers connected to the same pathology represents an important tool for attaining a higher accuracy of the assay of interest. To that end, the inventors observed a significant increase in the discriminating power of the assay of the invention when the values related to anti-ezrin antibodies measured in blood from affected subjects and controls, scored according to a predetermined cut-off value (preferably equal to 0.1183 optical density arbitrary units, O.D.), are used in combination with data related to serum CA 19.9 antigen concentrations measured in the same subjects (cut-off value preferably equal to 37 IU/ml). Indeed, as deduced from analysis of the ROC curve illustrated in Figure 3B and designed on the basis of the combination of the abovementioned measurements, the value of the area under the curve (AUC) undergoes a significant increase to 0.92. In order to further strengthen the diagnostic significance of the method of the invention, also the use of another biomarker already known as an indicator of pancreatic neoplasia, more specifically the presence of circulating anti-phosphorylated ENOA1-2 auto-antibodies, in addition to or as an alternative to the assessment of the CA 19.9 antigen, combined with determination of anti-ezrin auto-antibodies, proved to be an important aid in achieving a more accurate grading of the clinical samples tested, particularly when the measured values of the levels of anti-ezrin auto-antibodies and CA 19.9 antigen are discordant, thereby allowing to reach a diagnostic accuracy equal to 0.97±0.02 (100% sensitivity, 93.7% specificity) (Figure 3C).

Therefore, in a preferred embodiment, the method of the invention comprises, in combination with assessment of anti-ezrin auto-antibodies, determining the presence, absence or level of CA 19.9 antigen and/or the presence, absence or level of antibodies directed towards phosphorylated alpha-enolase.

Among the most largely used methods for identifying and quantifying an antigen or alternatively an antibody specific against the said antigen in a biological sample, the immunological procedures are the method of choice as, because they rely on the formation of an antigen-antibody complex, they allow to obtain a high degree of specificity. Hence, in a preferred embodiment, the method of the invention is an immunoassay.

Preferably, assessment of the anti-ezrin auto-antibodies according to the method of the invention is carried out by using an affinity reagent, preferably proteinaceous or peptidic in nature, suitable for selectively binding the abovementioned antibodies. In a preferred embodiment, the affinity reagent is the human ezrin protein or a fragment thereof, such as for example an ezrin peptide.

In another preferred embodiment, the method of the invention further comprises using an affinity reagent capable of detecting in the test sample the presence of the CA 19.9 antigen and/or an affinity reagent capable of detecting in the test sample the presence of auto-antibodies against phosphorylated ENOA1-2. For instance, the use of monoclonal or polyclonal antibodies able to specifically bind the carbohydrate 19.9 antigen or protein or peptidic antigens capable of capturing circulating antibodies directed towards phosphorylated alpha-enolase is suitable to that end. However, the method of the invention is not restricted to the use of monoclonal or polyclonal antibody molecules, but can use any affinity reagent suitable for such aim, for example an aptamer, a monomeric (Fab) or dimeric (F(ab')2) antibody fragment, a single chain antibody fragment (scFv), or any binding protein derived from an antibody or non-antibody scaffold; non-limiting examples of non-antibody scaffolds are fibronectin 3, protein A, lipocalin, tetranectin protein, ankyrin domains, etc. The selection of the most suitable affinity reagent to be used in the method of the invention falls within the knowledge and skills of the ordinary person of skill in the art.

In a preferred embodiment, a secondary antibody capable of selectively binding the immunocomplex formed between the antigen and the antibody under examination is used in order to increase the specificity of an immunoassay. Typically, the secondary antibody is directed towards the antibody that is part of the target immunocomplex, the so-called primary antibody, and is generated in an animal species that is different from the one from which the primary antibody was derived. In an alternative embodiment, the secondary antibody binds to an epitope on the antigen molecule which is different from the epitope specifically recognized by the primary antibody. Therefore, in a preferred embodiment, the method of the invention is an immunoassay wherein at least one secondary antibody, as defined above, is used.

The secondary antibody used in the method of the invention is labeled with a detectable label preferably selected from a radioactive isotope, a fluorophore, an enzyme, a chemiluminescent compound, an affinity label such as for example avidin or biotin. Fluorescein isothiocyanate and tetramethyl rhodamine are mentioned by way of example among the fluorescent labels most commonly used in immunoassays, the which are able to give the labeled compound stability and strength of the signal that is generally measurable in real time. Radioiodine, which can be incorporated in the protein chain tyrosine residues or added to the protein chain in the form of a pre-labeled tyrosine residue, is largely used in the radioimmunoassay field. Instead, the immunoenzyme methods are based on the detection of the antigen/antibody recognition immunological reaction through a reaction on a suitable substrate catalyzed by an enzyme chemically conjugated to the antibody molecule, for instance alkaline phosphatase and horseradish peroxidase, resulting in production of a colored signal. In the immunoenzyme methods, the signal amplification effect due to transformation of the substrate allows to detect trace amounts of analytes in the test sample.

Obviously, when the method of the invention seeks the determination of one or more other biomarkers in addition to anti-ezrin auto-antibodies, that is CA 19.9 antigen and optionally anti-phosphorylated ENOA antibodies, and thus involves the formation of two different immunocomplexes or three different immunocomplexes, the respective secondary antibodies optionally used in the assay are each labeled with a different detectable label, in order to allow to distinguish between the aforesaid immunocomplexes. The detectable labels differ among each other in the nature of the emitted detection signal, for example a chemiluminescent or fluorescent one, or in the range of the same type of signal, in other distinctive features such as for example the emission/absorption spectrum or the coloring produced. The selection of the most suitable detectable label to be used in the method of the invention falls well within the skills of the ordinary person of skill in the art.

In a preferred embodiment, the method of the invention is an Enzyme-Linked Immunosorbent Assay, known as ELISA. This kind of assay has a primary role in the field of immunoenzyme methods and is characterized by formation of an immunocomplex on a solid phase, generally a multiwell plate, by immobilization thereon of a capture antigen or antibody depending on whether the target ligand of the assay is an antibody or an antigen, respectively. The subsequent addition of a second antibody conjugated to an enzyme leads to formation of a so-called "sandwich" complex, the presence of which is detected by adding the chromogenic substrate and development of the color in the well.

In an alternative embodiment, the method of the invention is a Western blot immunoassay, a technique commonly used for identifying a protein in a complex protein mixture that has been subjected to analytic separation by polyacrylamide gel electrophoresis. After the separation, the proteins are transferred onto a support membrane, commonly a nitrocellulose one, and the protein molecules on the membrane can be identified by an immune mediated reaction using a labeled antibody capable of specifically recognizing the protein of interest.

Obviously, the use of any type of immunoassay format, the selection of which falls within the skills of the ordinary person of skill in the art, is within the scope of the present invention.

The means suitable for performing the method of the invention are assembled into a kit in order to facilitate the use thereof. Therefore, a further object of the invention is a kit for *in vitro* diagnosis of pancreatic ductal adenocarcinoma, as defined in claim 9 and in claim 10.

The kit of the invention comprises one affinity reagent, preferably a protein or peptide antigen, capable of binding anti-ezrin antibodies, and at least one further affinity reagent capable of recognizing CA 19.9 antigen and/or auto-antibodies against phosphorylated ENOA1-2, as well as the relevant detection means. The reagents contained in the kit are as defined above in connection with the method.

In a preferred embodiment, the kit of the invention further comprises a solid support, preferably selected from a multiwell plate, microsphere, membrane, slide, resin, sensor chip, bead. Such a support is used, for instance, for immobilizing one or more antigen and/or antibody molecules. Polyvinyl or polystyrene plates containing 96 or 384 wells, which allow for the simultaneous analysis of a large number of samples, are generally used in the common practice. Moreover, the materials the multiwell plates are made of favor a passive absorption of macromolecules through electrostatic bonds and hydrophobic interactions. Alternatively, the solid support of the kit of the invention is a microsphere, for instance a polystyrene or Sephadex microsphere, on the surface of which the immunocomplex formation and the immunometric assay take place. The microspheres of the kit of the invention may display functionalized surfaces, for example through coating with antibodies, or they may be magnetic microspheres in order to facilitate the separation thereof from the solution in which they are suspended by using a magnet. The selection of the most suitable solid support to be used in the kit of the invention falls well within the skills of the ordinary person of skill in the art.

The examples that follow are provided by way of illustration and not by way of limitation of the scope of the invention as defined in the appended claims.

### EXAMPLE 1: Murine pancreatic ductal adenocarcinoma model and collection of serum samples

The animals used in the experiments were treated in accordance with the government and European guidelines (legislative decree No. 116/92). 129SvJae H-2D^{b} mice that express the two mutated Kras^{G12D} and Trp53^{R172H} oncogenes under the control of the endogenous promoter and wherein, upstream of the first exon, a stop cassette is present, which is flanked by lox P sites (LSL) (*LSL-Kras*^{*G12D*/+}*, LSL-Trp53*^{*R172H*/+})*,* were kindly provided by Dr. David Tuveson (Cancer Research UK, Cambridge Research Institute, Cambridge, United Kingdom). C57BL/6 mice that express the Cre recombinase enzyme under the control of the pancreas-specific Pdx1 promoter (*Pdx-1-Cre*) were kindly provided by Dr. Andrew Lowy (University of San Diego, San Diego, CA, United States of America). The two strains *LSL-Kras*^{*G12D*/+}*, LSL-Trp53*^{*R172H*/+} and *Pdx-1-Cre* were crossbred in order to obtain the *LSL-Kras*^{*G12D*/+}*; LSL-Trp53*^{*R172H*/+}*; Pdx-1-Cre* (KPC) model (S. R. Hingorani et al., 2003 Cancer Cell 4, 437; S. R. Hingorani et al., 2005 Cancer Cell 7, 469-83). In order to take serum samples from the animals, the mice were sacrificed and the blood collected by intracardiac puncture. The presence of pancreatic tumor and possible metastases was confirmed by anatomo-pathological analysis of the sacrificed mice.

### EXAMPLE 2: Collection of serum samples from patients and controls

This study was approved by the ethical committee of the "Ospedale Universitario San Giovanni Battista, Università di Torino", Turin (authorization No. 54810). The serum samples were isolated from venous blood taken, at the time of diagnosis, from patients affected by ACP and healthy donors, with their informed consent. The serum samples were divided into aliquots and stored at -80°C. For each clinical case, the pancreatic ductal adenocarcinoma diagnosis was confirmed by histological or cytological analysis. In order to examine the humoral response evoked by ACP onset, 120 sera from patients affected by ACP (M/F: 67/53; average age, 67 years old; age range, 32-86 years old), the clinical characteristics of which were previously described in Tomaino B, et al. CIRCULATING AUTOANTIBODIES TO PHOSPHORYLATED ALPHA-ENOLASE ARE A HALLMARK OF PANCREATIC CANCER. J Proteome Res. 2011; 10:105-112, were analyzed by the SERPA method and 69 sera by the EISA approach. The immunoreactivity in the tested samples was compared to that of the following control sera: 60 sera taken from healthy subjects without a history of cancer or autoimmune diseases (HS, M/F: 25/35; average age, 70 years old; age range, 49-90 years old); 50 sera from patients affected by other neoplasia types (9 patients with hepatocarcinoma, 12 with mammary carcinoma, 9 with colorectal cancer, 19 with lung cancer, and 1 with ovary cancer; M/F: 24/26; average age: 69 years old; age range: 44-86 years old); 46 sera taken from patients with chronic pancreatitis (CP, M/F: 26/20; average age 58 years old; age range: 22-74 years old), and 12 sera from patients affected by autoimmune diseases (AD, 3 with mixed cryoglobulinemia, 2 with Meniere's syndrome, 4 with rheumatoid arthritis, 2 with systemic lupus erythematosus, and 1 with autoimmune pancreatitis; M/F: 3/9; average age: 49 years old; age range: 38-79 years old).

In order to verify the predictive value of the assay of the invention, serum samples taken before the appearance of pancreatic ductal adenocarcinoma symptoms and the consequent diagnosis of the disease were also included in this study. The aforesaid samples and the matched controls were obtained from the Turin cohort of the EPIC (European Prospective Investigation into Cancer and Nutrition) study including samples from 10604 healthy donors at the time of enrolment (6047 males and 4557 females, age 35-65 years old). The recruitment took place between 1993 and 1998 and involved blood donors and healthy volunteers. The cooperation with the local cancer registries and the local health authorities allowed for the monitoring over time of the onset of different cancer types and other diseases as well as deaths. The study design, population, and collected data have been described in detail previously (Riboli E et al, Public Health Nutrition (2002), 5: p. 1113-1124). In the extent of the EPIC study, controls are represented by enrolled donors that developed no tumor type or autoimmune disease during the follow-up period. They were selected and matched on the basis of different parameters, including the collection center, age, sex, date of enrolment and non-enrolment in the study. For the EPIC study too, the collection of the samples took place with the informed consent of the participants in the study and with the approval of the local ethical committees.

### EXAMPLE 3: Two-dimensional electrophoresis and Western blot analysis

In order to examine the immunoreactivity specifically induced by onset and progression of pancreatic carcinoma both in mice and human patients, serum samples taken from the same were assayed on extracts from pancreatic cancer cells. Particularly, two different pancreatic ductal adenocarcinoma cell lines were used in this study: the human CF-PAC-1 line (ECACC No. 91112501) and the murine K8484 line (isolated from a *KPC* mouse and kindly given by Dr. Tuveson, Cambridge University). The cells of the two lines were cultured in DMEM medium in the presence of 10% fetal bovine serum (FBS), glutamine (20mM) and gentamycin (50 µg/ml). The cell cultures were maintained in an incubator set at 37°C, with a 5% CO₂ humidified atmosphere. At a suitable growth stage, the cells were collected, washed with PBS, isolated from the culture medium by centrifugation and stored at -80°C. For each of the two cell lines used in this study, 10⁷ cells were resuspended in 1 ml of lysis buffer composed of urea (5 M), thiourea (2 M), CHAPS (4% w/v), Na₃VO₄ (1 mM), PMSF (1 mM), DTT (80 mM), protease inhibitor mix (10 µl/ml) and Nuclease Mix (10 µl/ml). The sample was left for 1 hour in ice and then sonicated (Hielscher UP200S, 3 × 40 s, amplitude 40%, cycles 0.5). After centrifugation at 13200 rpm/min for 30 min, the supernatant was collected and the protein concentration thereof was determined with the Bradford assay.

Additionally, eight samples of tumor tissue taken from patients affected by APC who underwent surgical resection (tumor stage II) were examined. The aforesaid tissue samples were obtained from the "Dipartimento di Chirurgia e Gastroenterologia" of the University of Verona. For each sample, a total of 30-50 mg of tissue was homogenized on ice in lysis buffer as described above.

The protein content of the cell lysates obtained according to the previously illustrated procedures was separated by isoelectric focusing using 7 cm-long polyacrylamide strips (IPG strip) with a non-linear pH gradient comprised between 3 and 10. For the purpose of obtaining analytical and preparative gels, each strip was loaded with 100 µg of protein lysate resuspended in a rehydration buffer composed of urea (5 M), thiourea (2 M), CHAPS (4% w/v), IPG buffer 3-10 NL (2% w/v), DTT (80 mM) and traces of Bromophenol Blue. The proteins were separated with a 5000V voltage gradient, for a total of 16000Vh. The isoelectric focusing was carried out by using the IPGphor Isoelectric Focusing System (GE Healthcare Bio-Sciences GmbH, Uppsala, Sweden). Prior to SDS-PAGE, the strips were reduced for 15 min with a buffer (Tris/HCl [50 mM; pH 8.8], urea [6 M], glycerol [30% v/v], SDS [2% w/v], and Bromophenol Blue) containing DTT (2% w/v) and then alkylated for 5 min in the same solution containing iodacetamide (2.5% w/v) instead of DTT. The SDS-PAGE was run on a NuPAGE® Novex® 4-12% Bis-Tris Zoom® mini-gel using the Novex X-Cell II™ Mini-cell system (Invitrogen, Groningen, the Netherlands) at a constant 200V. The thus obtained two-dimensional map was stained with colloidal Coomassie Brilliant Blue G-250 or transferred onto a nitrocellulose membrane for serological analysis. The isoelectric point values of the protein spots were estimated according to their position on the two-dimensional gel with pH gradient graphs provided by GE Healthcare Bio-Sciences GmbH. The molecular weight of the proteins was calculated by comparison with the migration of a protein mixture of known molecular weight, the SeeBlue Plus2 Pre-Stained Standard.

The preparative gels for mass spectrometry were first washed with deionized water and then left for 14 hours at 4°C in a 40% methanol and 10% acetic acid solution. Finally, they were stained with a mixture composed of Coomassie Brilliant Blue G-250 (1 liter of solution: 1.5 g Coomassie Brilliant Blue G-250, 100g Ammonium sulfate and 23.6 ml Phosphoric Acid) with 20% methanol. The images of the two-dimensional gels were acquired with the ProXPress 2D Proteomic Imaging System scanner (PerkinElmer, Boston, MA, United States of America) and stored in TIFF format with the ProScan 4.0 program (PerkinElmer, Boston, MA, United States of America).

For serological analysis, the maps of the two lines (CF-PAC-1 and K8484) were transferred onto nitrocellulose Hybond ECL membranes with the Novex X-Cell II™ Blot Module system (Invitrogen, Groningen, the Netherlands) at a constant 30V. After incubation for 1 hour with TBS (Tris-HCl 20 mM pH 7.5, NaCl 500 mM) containing 5% nonfat dry milk, the membranes were incubated at 4°C for 14 hours with the sera (dilution: 0.1 mg IgG/ml) in TBS with 0.05% Tween 20 (TTBS) and 5% nonfat dry milk. Each membrane containing the CFPAC-1 extract was incubated with one single human serum, similarly each membrane containing the K8484 extract was incubated with one single murine serum. After three 15-min washes with TTBS, the membranes were incubated for 90 min at room temperature with a rabbit anti-human immunoglobulin G (IgG) antibody (1:1000) or with a sheep anti-murine immunoglobulin G (IgG) antibody (1:5000), both conjugated to horseradish peroxidase and diluted in TTBS. Serum reactivity was detected by chemifluorescence reaction using ECL Plus. Images were acquired with the ProXPress 2D Proteomic Imaging System scanner (PerkinElmer) at an excitation wavelength of 460 nm and an emission wavelength of 530 nm and were stored in TIFF format with the ProScan 4.0 program (PerkinElmer). The Western blot images were then compared with those from the respective CF-PAC-1 or K8484 gels using the ProFinder 2D program (PerkinElmer). By such analyses, the serum-recognized proteins were located on the corresponding stained gels, then followed by identification through mass spectrometry.

### EXAMPLE 4: Protein identification by mass spectrometry

The proteins of interest were extracted from the preparative CF-PAC-1 and K8484 gels and destained for 15 hours with a solution of 5 mM ammonium bicarbonate and 50% acetonitrile. Then, the proteins were digested in gel with trypsin and analyzed by Matrix-Assisted Laser Desorption Ionization/Time of Flight (MALDI-TOF) mass spectrometry. For analysis by MALDI-TOF MS, 1,5 µl of each peptide mixture were added to 1,5 µl of matrix solution (1% w/v α-cyano-4-hydroxycinnamic acid in 40% acetonitrile and 60% 0.1% TFA) and applied to a target plate allowed to air-dry.

The spectra were obtained by using a Bruker Reflex III MALDI-TOF spectrometer (Bremen, Germany). The spectra interpretation of the tryptic digests was done by peptide mass fingerprinting (PMF) using the MassScot program *(www.matrixscience.com).*

### EXAMPLE 5: ELISA assay

Ezrin protein was immobilized (0.5 µg/ml in PBS) in 96-well microplates overnight at room temperature; then the free sites were blocked with PBS containing 4% bovine serum albumin for 2 hours at room temperature. Patients' sera (0.01 mg/ml dilution) diluted in PBS, 1% BSA, 0.05% Tween-20 were added to the wells and incubated for 2 hours at room temperature. After the washes, the microplates were incubated with the rabbit anti-human immunoglobulin G (IgG) antibody conjugated to horseradish peroxidase (HRP) (1:1000 dilution) for 1 hour at room temperature; then, TMB was added to each well. The reaction was stopped with 2N HCl and the optical density (OD) value was measured at 450 nm. All samples were analyzed in triplicate and the results represent the mean values from which the background, detected in wells in which the protein had not been immobilized, was subtracted.

### EXAMPLE 6: Statistical analysis

The statistical analysis was performed with the GraphPad (Version 4, San Diego, CA), MedCalc (Version 11.4.2.0, Mariakerke, Belgium), and SPSS (Version 18.0, Chicago, IL, United States of America) programs. Age and sex distribution of patients and controls was analyzed with the Student's t-test and Fisher's test, respectively. The statistical analysis of the recognition frequencies of tumor antigens by sera from patients and controls was performed by Fisher's test in the SERPA analysis and by Student's t-test in the ELISA analysis. The ROC (Receiver Operating Characteristics) curve was designed in order to establish the optimum threshold values that were able to distinguish patients from controls. Correlations and associations between variables were analyzed by Pearson's, Chi-square, or Fisher's tests, as most appropriate. In all tests, P < .05 (*), P < .005 (**) and P < .0005 (***) values were considered to be statistically significant.

## Claims

1. A method for the *in vitro* diagnosis of pancreatic ductal adenocarcinoma in a subject or for determining the predisposition of a subject to pancreatic ductal adenocarcinoma, comprising the step of determining in a biological sample from the subject, the presence, the absence or the level of antibodies against ezrin, wherein the presence of antibodies against ezrin or the level of antibodies against ezrin above a predetermined threshold is indicative of pancreatic ductal adenocarcinoma or of a predisposition to pancreatic ductal adenocarcinoma.

2. The method according to claim 1, wherein the step of determining the presence, the absence or the level of antibodies directed against ezrin is performed by an immunoassay, preferably by the use of ezrin as an affinity reagent suitable to bind antibodies against ezrin thereby forming a first immunocomplex.

3. The method according to claim 2, wherein the affinity reagent is human ezrin.

4. The method according to any of claims 1 to 3, further comprising the step of determining the presence, the absence or the level of carbohydrate antigen 19.9 in the biological sample from the subject.

5. The method according to claim 4, wherein the step of determining the presence, the absence or the level of carbohydrate antigen 19.9 is performed by an immunoassay, preferably by the use of an antibody against carbohydrate antigen 19.9 as an affinity reagent suitable to bind carbohydrate antigen 19.9, thereby forming a second immunocomplex.

6. The method according to any of claims 1 to 5, further comprising the step of determining the presence, the absence or the level of antibodies directed against phosphorylated alpha-enolase in the biological sample from the subject.

7. The method according to claim 6, wherein the step of determining the presence, the absence or the level of antibodies directed against phosphorylated alpha-enolase is performed by an immunoassay, preferably by the use of a phosphorylated alpha-enolase protein or a peptidic antigen thereof that is able to capture circulating antibodies directed against phosphorylated alpha-enolase protein as an affinity reagent suitable to bind antibodies directed against phosphorylated alpha-enolase, thereby forming a third immunocomplex.

8. The method according to any of claims 1 to 7, which is an ELISA immunoassay or a Western Blot immunoassay.

9. A kit for the *in vitro* diagnosis of pancreatic ductal adenocarcinoma in a subject or for determining the predisposition of a subject to pancreatic ductal adenocarcinoma, comprising:
- at least one affinity reagent suitable to bind antibodies against ezrin thereby forming a first immunocomplex, wherein the affinity reagent suitable to bind antibodies against ezrin is ezrin;
- at least one further affinity reagent selected from an affinity reagent suitable to bind carbohydrate antigen 19.9 thereby forming a second immunocomplex, and an affinity reagent suitable to bind antibodies against phosphorylated alpha-enolase thereby forming a third immunocomplex,
wherein the affinity reagent suitable to bind carbohydrate antigen 19.9 is an antibody against carbohydrate antigen 19.9,
and wherein the affinity reagent suitable to bind antibodies against phosphorylated alpha-enolase is a phosphorylated alpha enolase protein or a peptidic antigen thereof that is able to capture circulating antibodies directed against phosphorylated alpha-enolase protein;
- means for detecting the first immunocomplex; and
- means for detecting the second immunocomplex or means for detecting the third immunocomplex.

10. The kit according to claim 9, comprising:
- at least one affinity reagent suitable to bind antibodies against ezrin thereby forming a first immunocomplex, wherein the affinity reagent suitable to bind antibodies against ezrin is ezrin;
- at least one affinity reagent suitable to bind carbohydrate antigen 19.9 thereby forming a second immunocomplex; and
- at least one affinity reagent suitable to bind antibodies against phosphorylated alpha-enolase thereby forming a third immunocomplex,
wherein the affinity reagent suitable to bind carbohydrate antigen 19.9 is an antibody against carbohydrate antigen 19.9,
and wherein the affinity reagent suitable to bind antibodies against phosphorylated alpha-enolase is a phosphorylated alpha enolase protein or a peptidic antigen thereof that is able to capture circulating antibodies directed against phosphorylated alpha-enolase protein,
- means for detecting the first immunocomplex;
- means for detecting the second immunocomplex; and
- means for detecting the third immunocomplex.

11. The kit according to any of claims 9 to 10, wherein the means for detecting the first immunocomplex comprise a secondary antibody suitable to bind the first immunocomplex, wherein the secondary antibody is preferably labeled with a first detectable label preferably selected from the group consisting of enzyme labels, chemiluminescent labels, fluorescent labels, radioactive labels, affinity labels.

12. The kit according to any of claims 9 to 11, wherein the means for detecting the second immunocomplex comprise a secondary antibody suitable to bind the second immunocomplex, wherein the secondary antibody is preferably labeled with a second detectable label preferably selected from the group consisting of enzyme labels, chemiluminescent labels, fluorescent labels, radioactive labels, affinity labels.

13. The kit according to any of claims 9 to 12, wherein the means for detecting the third immunocomplex comprise a secondary antibody suitable to bind the third immunocomplex, wherein the secondary antibody is preferably labeled with a third detectable label which is preferably selected from the group consisting of enzyme labels, chemiluminescent labels, fluorescent labels, radioactive labels, affinity labels.

## Patentansprüche

1. Verfahren für die In-vitro-Diagnostik eines duktalen Pankreaskarzinoms bei einem Probanden oder für das Bestimmen der Veranlagung eines Probanden für ein duktales Pankreaskarzinom, umfassend einen Schritt des Erfassens der Anwesenheit, Abwesenheit oder des Gehalts an Ezrin-Antikörpern in einer biologischen Probe des Probanden, wobei die Anwesenheit von Antikörpern gegen Ezrin oder der Gehalt an Ezrin-Antikörpern über einen vorab festgelegten Grenzwert als Indikator für ein duktales Pankreaskarzinom oder für eine Veranlagung für ein duktales Pankreaskarzinom dient.

2. Verfahren nach Anspruch 1, wobei der Schritt des Erfassens der Anwesenheit, Abwesenheit oder des Gehalts an Ezrin-Antikörpern in Form eines Immunassays stattfindet, vorzugsweise unter Verwendung von Ezrin als Affinitätsreagenz, geeignet Antikörper gegen Ezrin zu binden und so einen ersten Immunkomplex zu bilden.

3. Verfahren nach Anspruch 2, wobei das Affinitätsreagenz Human-Ezrin ist.

4. Verfahren nach einem der Ansprüche 1 bis 3, des Weiteren einen Schritt des Erfassens der Anwesenheit, Abwesenheit oder des Gehalts an Carbohydrate-Antigen 19-9 in einer biologischen Probe des Probanden umfassend.

5. Verfahren nach Anspruch 4, wobei der Schritt des Erfassens der Anwesenheit, Abwesenheit oder des Gehalts an Carbohydrate-Antigen 19-9 in Form eines Immunassays stattfindet, vorzugsweise unter Verwendung eines Antikörpers gegen das Carbohydrate-Antigen 19-9 als Affinitätsreagenz, geeignet Carbohydrate-Antigen 19-9 zu binden und so einen zweiten Immunkomplex zu bilden.

6. Verfahren nach einem der Ansprüche 1 bis 5, des Weiteren einen Schritt des Erfassens der Anwesenheit, Abwesenheit oder des Gehalts an Antikörpern gegen phosphorylierte alpha-Enolase in einer biologischen Probe des Probanden umfassend.

7. Verfahren nach Anspruch 6, wobei der Schritt des Erfassens der Anwesenheit, Abwesenheit oder des Gehalts an Antikörpern gegen phosphorylierte alpha-Enolase in Form eines Immunassays stattfindet, vorzugsweise unter Verwendung eines phosphorylierten alpha-Enolase-Proteins oder eines Peptidantigens davon, das in der Lage ist, zirkulierende Antikörper gegen phosphorylierte alpha-Enolase-Proteine als Affinitätsreagenz zu erfassen, geeignet Antikörper gegen phosphorylierte alpha-Enolase zu binden und so einen dritten Immunkomplex zu bilden.

8. Verfahren nach einem der Ansprüche 1 bis 7, wobei es sich um einen ELISA-Immunassay oder einen Western-Blot-Immunassay handelt.

9. Ein Set für die In-vitro-Diagnostik eines duktalen Pankreaskarzinoms bei einem Probanden oder für das Bestimmen der Veranlagung eines Probanden für ein duktales Pankreaskarzinom, umfassend:
- mindestens ein Affinitätsreagenz, das geeignet ist, Antikörper gegen Ezrin zu binden und so einen ersten Immunkomplex zu bilden, wobei das Affinitätsreagenz geeignet zum Binden von Ezrin-Antikörpern Ezrin ist;
- mindestens ein weiteres Affinitätsreagenz, ausgewählt aus einem Affinitätsreagenz, das geeignet ist, Carbohydrate-Antigen 19-9 zu binden und so einen zweiten Immunkomplex zu bilden, sowie ein Affinitätsreagenz, das geeignet ist, Antikörper gegen phosphorylierte alpha-Enolase zu binden und so einen dritten Immunkomplex zu bilden;
wobei das Affinitätsreagenz, das geeignet ist Carbohydrate-Antigen 19-9 zu binden, ein Antikörper gegen Carbohydrate-Antigen 19-9 ist
und wobei das Affinitätsreagenz, das geeignet ist Antikörper gegen phosphorylierte alpha-Enolase zu binden, ein phosphoryliertes alpha-Enolase-Protein oder ein Peptidantigen davon ist, das in der Lage ist, zirkulierende Antikörper gegen phosphorylierte alpha-Enolase-Proteine zu erfassen;
- Mittel zum Erfassen des ersten Immunkomplexes sowie
- Mittel zum Erfassen des zweiten Immunkomplexes oder Mittel zum Erfassen des dritten Immunkomplexes.

10. Set nach Anspruch 9, umfassend:
- mindestens ein Affinitätsreagenz, das geeignet ist, Antikörper gegen Ezrin zu binden und so einen ersten Immunkomplex zu bilden, wobei das Affinitätsreagenz geeignet zum Binden von Ezrin-Antikörpern Ezrin ist;
- mindestens ein Affinitätsreagenz, das geeignet ist, Carbohydrate-Antigen 19-9 zu binden und so einen zweiten Immunkomplex zu bilden, sowie
- mindestens ein Affinitätsreagenz, das geeignet ist, Antikörper gegen phosphorylierte alpha-Enolase zu binden und so einen dritten Immunkomplex zu bilden;
wobei das Affinitätsreagenz, das geeignet ist, Carbohydrate-Antigen 19-9 zu binden, ein Antikörper gegen Carbohydrate-Antigen 19-9 ist
und wobei das Affinitätsreagenz, das geeignet ist, Antikörper gegen phosphorylierte alpha-Enolase zu binden, ein phosphoryliertes alpha-Enolase-Protein oder ein Peptidantigen davon ist, das in der Lage ist, zirkulierende Antikörper gegen phosphorylierte alpha-Enolase-Proteine zu erfassen;
- Mittel zum Erfassen des ersten Immunkomplexes;
- Mittel zum Erfassen des zweiten Immunkomplexes sowie
- Mittel zum Erfassen des dritten Immunkomplexes.

11. Set nach einem der Ansprüche 9 bis 10, wobei die Mittel zum Erfassen des ersten Immunkomplexes einen Sekundärantikörper umfassen, geeignet den ersten Immunkomplex zu binden, wobei der Sekundärantikörper vorzugsweise mit einer ersten nachweisbaren Markierung markiert ist, vorzugsweise ausgewählt aus einer Gruppe bestehend aus Enzymmarkierungen, Chemielumineszenzmarkierungen, Fluozeszenzmarkierungen, radioaktiven Markierungen, Affinitätsmarkierungen.

12. Set nach einem der Ansprüche 9 bis 11, wobei die Mittel zum Erfassen des zweiten Immunkomplexes einen Sekundärantikörper umfassen, geeignet den zweiten Immunkomplex zu binden, wobei der Sekundärantikörper vorzugsweise mit einer zweiten nachweisbaren Markierung markiert ist, vorzugsweise ausgewählt aus einer Gruppe bestehend aus Enzymmarkierungen, Chemielumineszenzmarkierungen, Fluozeszenzmarkierungen, radioaktiven Markierungen, Affinitätsmarkierungen.

13. Set nach einem der Ansprüche 9 bis 12, wobei die Mittel zum Erfassen des dritten Immunkomplexes einen Sekundärantikörper umfassen, geeignet den dritten Immunkomplex zu binden, wobei der Sekundärantikörper vorzugsweise mit einer dritten nachweisbaren Markierung markiert ist, vorzugsweise ausgewählt aus einer Gruppe bestehend aus Enzymmarkierungen, Chemielumineszenzmarkierungen, Fluozeszenzmarkierungen, radioaktiven Markierungen, Affinitätsmarkierungen.

## Revendications

1. Procédé de diagnostic *in vitro* de l'adénocarcinome canalaire pancréatique chez un sujet ou de détermination de la prédisposition d'un sujet à l'adénocarcinome canalaire pancréatique, comprenant l'étape de détermination, dans un échantillon biologique du sujet, de la présence, de l'absence ou du niveau d'anticorps contre l'ezrine, dans lequel la présence d'anticorps contre l'ezrine ou le niveau d'anticorps contre l'ezrine au-delà d'un seuil prédéterminé est indicatif d'un adénocarcinome canalaire pancréatique ou d'une prédisposition à l'adénocarcinome canalaire pancréatique.

2. Procédé selon la revendication 1, dans lequel l'étape de détermination de la présence, de l'absence ou du niveau d'anticorps dirigés contre l'ezrine est réalisée par un immunoessai, de préférence en utilisant l'ezrine en tant que réactif d'affinité approprié pour lier les anticorps contre l'ezrine, formant de ce fait un premier complexe immun.

3. Procédé selon la revendication 2, dans lequel le réactif d'affinité est l'ezrine humaine.

4. Procédé selon l'une des revendications 1 à 3, comprenant en outre l'étape de détermination de la présence, de l'absence ou du niveau d'antigène carbohydrate 19-9 dans l'échantillon biologique du sujet.

5. Procédé selon la revendication 4, dans lequel l'étape de détermination de la présence, de l'absence ou du niveau d'antigène carbohydrate 19-9 est réalisée par un immunoessai, de préférence par l'utilisation d'un anticorps contre l'antigène carbohydrate 19-9 en tant que réactif d'affinité approprié pour lier l'antigène carbohydrate 19-9, et former de ce fait un deuxième complexe immun.

6. Procédé selon l'une des revendications 1 à 5, comprenant en outre l'étape de détermination de la présence, de l'absence ou du niveau d'anticorps dirigés contre l'alpha-énolase phosphorylée dans l'échantillon biologique du sujet.

7. Procédé selon la revendication 6, dans lequel l'étape de détermination de la présence, de l'absence ou du niveau d'anticorps dirigés contre l'alpha-énolase phosphorylée est réalisée par un immunoessai, de préférence par l'utilisation d'une protéine alpha-énolase phosphorylée ou d'un antigène peptidique de celle-ci qui est capable de capturer les anticorps circulants dirigés contre la protéine alpha-énolase phosphorylée en tant que réactif d'affinité approprié pour lier les anticorps dirigés contre l'alpha-énolase phosphorylée, formant de ce fait un troisième complexe immun.

8. Procédé selon l'une des revendications 1 à 7, qui est un immunoessai ELISA ou un immunoessai de type Western Blot.

9. Kit de diagnostic *in vitro* de l'adénocarcinome canalaire pancréatique chez un sujet ou de détermination de la prédisposition d'un sujet à l'adénocarcinome canalaire pancréatique, comprenant :
- au moins un réactif d'affinité approprié pour lier des anticorps dirigés contre l'ezrine et former de ce fait un premier complexe immun, où le réactif d'affinité approprié pour lier les anticorps contre l'ezrine est l'ezrine ;
- au moins un autre réactif d'affinité sélectionné parmi un réactif d'affinité approprié pour lier l'antigène carbohydrate 19-9 et former de ce fait un deuxième complexe immun, et un réactif d'affinité approprié pour lier des anticorps contre l'alpha-énolase phosphorylée et former de ce fait un troisième complexe immun,
où le réactif d'affinité approprié pour lier l'antigène carbohydrate 19-9 est un anticorps dirigé contre l'antigène carbohydrate 19-9,
et où le réactif d'affinité approprié pour lier les anticorps dirigés contre l'alpha-énolase phosphorylée est une protéine alpha-énolase phosphorylée ou un antigène peptidique de celle-ci qui est capable de capturer des anticorps circulants dirigés contre la protéine alpha-énolase phosphorylée ;
- des moyens de détection du premier complexe immun ; et
- des moyens de détection du deuxième complexe immun ou des moyens de détection du troisième complexe immun.

10. Kit selon la revendication 9, comprenant :
- au moins un réactif d'affinité approprié pour lier des anticorps contre l'ezrine et former de ce fait un premier complexe immun, où le réactif d'affinité approprié pour lier les anticorps contre l'ezrine est l'ezrine ;
- au moins un réactif d'affinité approprié pour lier l'antigène carbohydrate 19-9 et former de ce fait un deuxième complexe immun ; et
- au moins un réactif d'affinité approprié pour lier les anticorps contre l'alpha-énolase phosphorylée et former de ce fait un troisième complexe immun,
où le réactif d'affinité approprié pour lier l'antigène carbohydrate 19-9 est un anticorps dirigé contre l'antigène carbohydrate 19-9,
et où le réactif d'affinité approprié pour lier les anticorps dirigés contre l'alpha-énolase phosphorylée est une protéine alpha-énolase phosphorylée ou un antigène peptidique de celle-ci qui est capable de capturer des anticorps circulants dirigés contre la protéine alpha-énolase phosphorylée,
- des moyens de détection du premier complexe immun ;
- des moyens de détection du deuxième complexe immun ; et
- des moyens de détection du troisième complexe immun.

11. Kit selon l'une des revendications 9 ou 10, dans lequel les moyens de détection du premier complexe immun comprennent un anticorps secondaire approprié pour lier le premier complexe immun, où l'anticorps secondaire est de préférence marqué avec un premier marqueur détectable, de préférence sélectionné dans le groupe composé de marqueurs enzymatiques, de marqueurs de chimiluminescence, de marqueurs fluorescents, de marqueurs radioactifs et de marqueurs d'affinité.

12. Kit selon l'une des revendications 9 à 11, dans lequel les moyens de détection du deuxième complexe immun comprennent un anticorps secondaire approprié pour lier le deuxième complexe immun, où l'anticorps secondaire est de préférence marqué avec un deuxième marqueur détectable sélectionné dans le groupe composé de marqueurs enzymatiques, de marqueurs de chimiluminescence, de marqueurs fluorescents, de marqueurs radioactifs et de marqueurs d'affinité.

13. Kit selon l'une des revendications 9 à 12, dans lequel les moyens de détection du troisième complexe immun comprennent un anticorps secondaire approprié pour lier le troisième complexe immun, où l'anticorps secondaire est de préférence marqué avec un troisième marqueur détectable sélectionné dans le groupe composé de marqueurs enzymatiques, de marqueurs de chimiluminescence, de marqueurs fluorescents, de marqueurs radioactifs et de marqueurs d'affinité.
